Europäisches Patentamt

European Patent Office

Office européen des brevets

(19)

(11) Publication number: **0 114 403**
**B1**

(12) **EUROPEAN PATENT SPECIFICATION**

(45) Date of publication of patent specification: **05.08.87**

(51) Int. Cl.⁴: **G 01 N 33/52, G 01 N 31/22**

(21) Application number: **83113147.9**

(22) Date of filing: **27.12.83**

(54) Multilayer analytical element.

(30) Priority: **28.12.82 JP 227980/82**

(43) Date of publication of application:
**01.08.84 Bulletin 84/31**

(45) Publication of the grant of the patent:
**05.08.87 Bulletin 87/32**

(84) Designated Contracting States:
**DE GB**

(56) References cited:
**DE-A-3 021 166**
**DE-A-3 029 301**
**DE-A-3 235 658**
**GB-A-2 085 159**
**GB-A-2 085 581**
**US-A-4 153 668**

(73) Proprietor: **FUJI PHOTO FILM CO., LTD.**
**210 Nakanuma Minami Ashigara-shi**
**Kanagawa 250-01 (JP)**

(72) Inventor: **Katsuyama, Harumi**
**c/o FUJI PHOTO FILM CO. LTD. 3-11-46 Senzi**
**Asaki-shi Saitama (JP)**
Inventor: **Kondo, Asaji**
**c/o FUJI PHOTO FILM CO. LTD. 3-11-46 Senzui**
**Asaka-shi Saitama (JP)**

(74) Representative: **Kraus, Walter, Dr. et al**
**Patentanwälte Kraus, Weisert & Partner**
**Thomas-Wimmer-Ring 15**
**D-8000 München 22 (DE)**

EP 0 114 403 B1

## Description

Field of the invention

The present invention relates to a multilayer analytical element, and more particularly to a multilayer analytical element suitable for analysis of a low diffusive substance contained in body fluids.

Description of prior arts

As a method of analysis of substance contained in a liquid sample such as a body fluid in a small amount, there has been conventionally utilized a wet analysis such as the method comprising the steps of bringing said substance into contact with other substances contained in a solvent so as to undergo a detectable reaction such as a reaction directly or indirectly producing or changing color, and detecting said reaction.

However, a dry analysis developed for the purpose of simplifying the analytical procedures has been recently utilized. As a representative example of the well-known analytical elements utilized for the dry analysis, is a multilayer analytical element (also called a multilayer analytical material or article) which is in the form of a sheet, film, strip or tape basically comprising at least one reagent layer containing a reagent which directly or indirectly undergoes a detectable reaction in contact with the substance under analysis (analyte), and a support therefor. The analysis employing the multilayer analytical element is generally carried out by applying a liquid sample containing the analyte onto the multilayer analytical element, subjecting the multilayer analytical element to incubation if necessary, measuring the thus produced detectable reaction such as the generation or change of color by means of photometry or the like, and determining the amount of analyte by colorimetry.

There are known a variety of analytical systems utilized in analysis of analytes employing multilayer analytical elements. Representative examples of the analytical systems are as follows.

(A) An analytical system for detecting and measuring a detectable reaction between a reagent contained in a reagent layer and an analyte such as the generation or change of color. The analytical system is employed for analysis of analyte such as a variety of proteins, for instance, total protein, albumin and globulin, a hemoglobin decomposition substance, for instance, free (non-conjugated or indirect) bilirubin or conjugated (direct) bilirubin.

(B) An analytical system employing a reagent layer containing at least two kinds of reagents, which comprises reacting one of the reagents with analyte to produce a reactive substance such as ammonia or hydrogen peroxide, subsequently reacting the reactive substance with the other reagent (e.g. dye precursor) in contact therebetween to cause a detectable reaction such as the generation or change of color, and detecting and measuring the reaction. The analytical system is employable for analysis where the analytes are glucose; lipids such as cholesterol, trigliceride and free fatty acid; enzymes such as lactate dehydrogenase; urea and uric acid.

(C) An analytical system employing a reagent layer containing an non-diffusive reagent having a color-forming group, which comprises converting the reagent into a diffusive product carrying the color-forming group by reaction with the analyte, separating the diffused product from the unreacted non-diffused reagent, causing a detectable reaction such as the generation or change of color in contact between the diffused product and a chromogenic substance such as a coupler, and detecting and measuring the reaction. The analytical system is employable for analysis of polysaccharide hydrolases such as amylase.

(D) An analytical system employing a reagent layer containing a non-diffusive reagent having a detectable property (e.g., color), which comprises producing a diffusive product carrying the detectable property from the reagent by reaction with an analyte, separating the diffused product from the unreacted non-diffused reagent, and detecting and measuring the diffused product. The analytical system is employable for analysis of polysaccharide hydrolases such as amylase.

The object of the present invention is to provide a multilayer analytical element employable for the above-exemplified analytical system (A), that is, an analytical system comprising detection of a detectable reaction between a reagent contained in a reagent layer and an analyte such as the generation or change of color.

The present invention provides a multilayer analytical element particularly suitable for analysis of high molecular weight analytes such as a variety of proteins e.g. total protein, albumin and globulin, or analytes such as hemoglobin decomposition substances e.g. free (unconjugated) bilirubin and conjugated bilirubin, as well as the analysis of hydrophobic analytes.

In more detail, the reagent layer of the known multilayer analytical element is generally made of a hydrophilic colloid such as a hydrophilic synthetic polymer or a hydrophilic natural polymer. In the reagent layer of the multilayer analytical element employed for the above-described purpose, the hydrophilic colloid layer contains a reagent producing a detectable reaction such as the generation or change of color in contact with the analyte (e.g. color precursor or dye). Accordingly, for carrying out the desired detectable reaction of analyte in the reagent layer within a short period of time upon introduction into the multilayer analytical element by applying a liquid sample onto the element, it is necessary that the analyte rapidly permeates and diffuses into the reagent layer so as to uniformly contact the reagent within a short period of time. However, it is difficult for high molecular weight analytes or hydrophilic analytes to permeate and

2

diffuse into the conventional reagent layer made of the hydrophilic colloids. Accordingly it is difficult to perform a rapid and accurate analysis.

Summary of the invention

It is, accordingly, an object of the present invention to provide a multilayer analytical element particularly suitable for analysis of analytes, having low diffusive properties into hydrophilic colloid layers such as various proteins, e.g. total protein, albumin and globulin, and hemoglobin decomposition substances, e.g., free bilirubin and conjugated bilirubin.

The present invention provides a multilayer analytical element for analysis of an analyte in a liquid sample comprising:

a porous spreading layer for spreading the liquid sample;

a porous reagent layer provided in fluid contact with said spreading layer which contains a reagent producing or changing color on reaction with the analyte; and

a liquid-impermeable, light-transmissive support supporting said reagent layer, which is characterized in that the porous reagent layer is a porous matrix comprising a binder containing a reagent producing or changing color on reaction with the analyte, and a particulate solid.

This invention also provides a multilayer analytical element for analysis of analyte present in a liquid sample comprising:

a porous spreading layer for spreading the liquid sample;

a porous reagent layer provided in fluid contact with said spreading layer which contains a reagent producing or changing color on reaction with the analyte;

a registration layer provided in fluid contact with said reagent layer which receives a color reaction product or a color-changed reaction product produced by the reaction between the analyte and the reagent; and

a liquid-impermeable, light-transmissive support supporting said registration layer, which is characterized in that the porous reagent layer is a porous matrix comprising a binder containing a reagent producing or changing color on reaction with the analyte, and a particulate solid.

Brief description of drawing

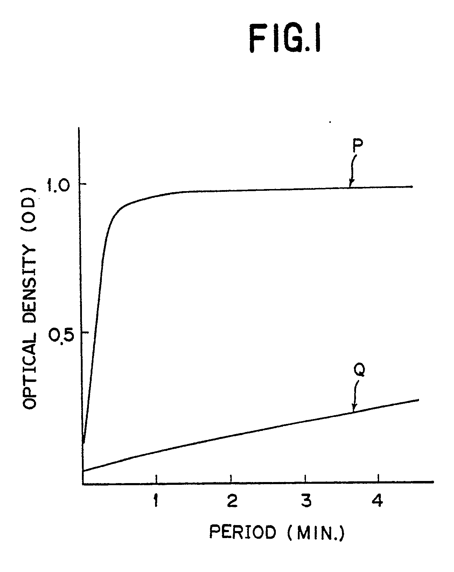

Fig. 1 illustrates curves showing the color formation rate of each multilayer analytical element in bilirubin analysis employing the multilayer analytical element of the present invention and the multilayer analytical element prepared for comparison.

P: Curve showing the color formation rate of the multilayer analytical element of the present invention

Q: Curve showing the color formation rate of the multilayer analytical element of Comparison Example 1

Detailed description of the invention

A multilayer analytical element composed mainly of a liquid-impermeable, light-transmissive support and a reagent layer of hydrophilic colloid containing a reagent producing or changing color on reaction with analyte has been conventionally employed in a variety of forms using various materials, and proposed for use. Also a multilayer analytical element composed mainly of a liquid-impermeable, light-transmissive support and single or plural reagent layers of hydrophilic colloids containing a reagent such as a color precursor or dye producing reaction such as generation or change of color in contact with an analyte has been conventionally employed in various forms using a variety of materials, and proposed for use.

Further it is known to enhance the analytical quantitativity by providing a porous spreading layer on a reagent layer, and a multilayer analytical element having such spreading layer is also known.

The liquid-impermeable, light-transmissive support and porous spreading layer of the multilayer analytical element of the present invention can be formed by utilizing the conventionally known materials and forms. Accordingly, the support and spreading layer constituting the multilayer analytical element of the present invention will be briefly described hereinafter.

A support employable for the multilayer analytical element is a support which a liquid, such as a liquid sample, does not permeate and light passes through. Such a support has the function of supporting a multilayer analytical element, as well as enabling the detection or measurement of the generation or change of color produced in the reagent layer by means of reflection photometry from the support side.

Examples of the support include organic resin films such as cellulose ester films (e.g. films of cellulose diacetate, cellulose triacetate and cellulose acetate propionate), polyethylene terephthalate films, films of polycarbonates of bisphenol A and polymethyl methacrylate films, and glass plates. There is no specific limitation to the thickness of the above-described support, but the thickness of the support is generally within the range of 50 µm to 2 mm, from the viewpoint of the aforementioned function.

The surface of the support can be provided with a known subbing layer, or subjected to a known chemical treatment such as acid treatment or alkali treatment or physico-chemical treatment such as corona discharge, glow discharge, irradiation with ultra-violet rays or flame treatment, for the purpose of facilitating the adhesion between the reagent layer (or other functional layer) and the support.

In the present invention, if the multilayer sheet comprising a reagent layer and a spreading layer is a self-supporting integral body, the reagent layer and/or spreading layer as such can serve as a support.

The spreading layer of the multilayer analytical element of the invention is basically located on the side of the reagent layer opposite to the light-transmissive support, and a liquid sample containing analyte is spotted onto the surface of the spreading layer in the analytical procedure. The spreading layer is a porous layer having the function of spreading the thus spotted liquid sample carrying the analyte over an area proportional to the amount of spotted liquid, and to supply uniformly a specific amount of the sample liquid per unit area to the reagent layer (referred to hereinafter as "spreading action" or "metering action"). Owing to the spreading action, the amount of the liquid supplied to the reagent layer through the spreading layer or the amount of analyte contained in the liquid to be supplied to the reagent layer is almost uniform per unit area of the reagent layer, even if the liquid sample is applied to the spreading layer in a varying amount. Accordingly, in the analysis of liquid sample employing the multilayer analytical element provided with a spreading layer having such a function, quantitative analysis can be made with high accuracy even if the liquid sample is applied to the spreading layer without accurately measuring the amount in the quantitative analysis. However, this fact does not mean that it is unnecessary to carry out the quantitative analysis by accurately measuring the amount of liquid sample in the procedure of analysis using the multilayer analytical element of the invention. The accuracy of the desired quantitative analysis can be further enhanced by accurately measuring the amount of liquid sample.

Examples of the porous spreading layer employable for the multilayer analytical element include a non-fibrous isotropic porous layer (e.g. a membrane filter or a blushed polymer layer), as described in Japanese Patent Provisional Publication No. 49(1974)—53888 (Japanese Patent Publication No. 53(1978)—21677) and U.S. Patent No. 3992158; an isotropic porous particulate structure layer containing continuous voids of three-dimensional lattice structure which is formed by the adhesion of fine spherical beads in point to point contact by an adhesive agent that is not swollen with water, as described in U.S. Patent 4,258,001; a fibrous anisotropic porous spreading layer made of fabric washed with water or a hydrophilically treated fabric, as described in U.S. Patent 4,292,272, compare also DE—A—3 021 166 and GB—A—2 052 057; a fibrous anisotropic porous spreading layer made of fabric having a physically activated surface, as described in GB—2,087,9074; and a fibrous anisotropic porous spreading layer made of paper, filter paper or nonwoven fabric containing synthetic polymer fibers, as described in Japanese Patent Provisional Publication No. 57(1982)—148250. The methods of providing these spreading layers on a multilayer analytical element are disclosed in each of the above-described publications, and these methods can be employed in the preparation of the multilayer analytical element of the present invention.

Examples of the matrix serving as a substrate of the porous reagent layer provided on the multilayer analytical element of the present invention include a non-fibrous isotropic porous layer (e.g. membrane filter or a blushed polymer), an isotropic porous particulate structure layer containing continuous voids of three-dimensional lattice structure which is formed through adhesion of fine spherical beads in point to point contact by an adhesive agent that is not swollen with water, a fibrous anisotropic porous layer made of fabrics, and a fibrous anisotropic porous layer made of paper, filter paper or nonwoven fabric containing synthetic polymer fibers.

The porous reagent layer is provided in fluid contact with the aforementioned spreading layer. "Fluid contact" means herein that the liquid sample applied to the surface of the spreading layer reaches the reagent layer carrying an analyte therein. The reagent layer is not necessarily provided in contact with · spreading layer, and other porous layers such as a filter layer may be provided between the spreading layer and the reagent layer in the form of single or plural layers.

The reagent layer provided in the multilayer analytical element of the present invention has a reagent participating in the analysis of the analyte contained in or attached to the above-described porous structure. The reagent employable in the multilayer analytical element of the invention produce or changes color on reaction with the analyte. Examples of reagents include a color precursor and a dye. These color precursors and dyes can be in various forms such as combinations with other low molecular weight compounds or high molecular weight compounds. The reagent layer of the multilayer analytical element can contain, as desired, a variety of additives such as surface active agents for the purpose of facilitating the uniformly dispersion of the reagent contained therein and substances for promoting the reaction between the reagent and the analyte.

The porous reagent layer of the present invention is a porous matrix comprising a binder containing a reagent producing or changing color through reaction with the analyte and a particulate solid. When a liquid sample having passed through a spreading layer contacts such a porous matrix, the liquid sample passes through the continuous space formed among the solid fine particles (particulate solid) and the binder in the porous matrix to rapidly reach the overall portion of the binder, and contacts the reagent contained in the binder to produce a color formation reaction or color change reaction.

There is no specific limitation to the particulate solid employable for formation of the above-described matrix, so long as the particulate solid does not give rise to an unfavorable effect on the multilayer analytical element of the invention by reaction between the analyte and the aforementioned various reagents. However, a transparent, opaque or white particulate solid is preferred. Examples of the particulate solid include a fine powder of silicon dioxide such as microcrystalline cellulose, diatomaceous earth or silica gel, a fine silicate powder such as natural and synthetic zeolite, or kaolin, fine particles of cellulose fibrous material, polymer beads, barium sulfate, and a metal oxide in the form of a fine white powder such as titanium dioxide, aluminium oxide or zinc oxide. There is no specific limitation to the form of the particulate solid, and any form such as spheres, columns, fibrous or pellets can be optionally employed.

When the photometry as reflection photometry is carried out under conditions such that the reaction product to be measured (colored product or color changed product) is present in the reagent layer containing the above-described particulate solids, the particulate solid included in the reagent layer containing the reaction product therein is preferably a low light-reflective particle so as not to reduce the accuracy of photometry. Examples of the particulate solid having low light reflectivity include microcrystalline cellulose, particulate diatomaceous earth, particulate silica gel, zeolite fine powder, particulate cellulose fiber, and polymer beads.

In the reagent layer of the multilayer analytical element of the present invention, the reaction product of the analyte and reagent such as a color precursor or dye can be subjected to detection such as by photometry with conditions that the reaction product remains in the reagent layer where the reaction is taken place.

However, depending upon the structure of the reagent layer or nature of the reaction between analyte and the reagent, the desired color formation reaction or color change reaction can proceed further rapidly and uniformly by transferring the reaction product to other layer provided separately from the reagent layer. In the use of the multilayer analytical element of the invention in such analytical system, there can be provided a registration layer for receiving the reaction product. The registration layer is provided in fluid contact with the reagent layer and receives the color formation product or color change product produced by the reaction between analyte and the reagent. "Fluid contact" means herein, as in the aforementioned case, that the reaction product in the reagent layer (reaction product showing a color or color change) reaches the registration layer with the liquid medium of the liquid sample or an independently introduced spreader liquid. Accordingly, the reagent layer and the registration layer are not necessarily provided in direct contact therebetween, and for instance, other porous layers such as a filter layer can be provided between the reagent layer and the registration layer in the form of single or plural layers.

The provision of the above-described registration layer is particularly advantageous when the analyte is bilirubin and the detecting agent is a diazonium compound therefor. In more detail, the bilirubin reacts with a diazonium compound to produce azobilirubin (azo dye) having its absorption spectral peak distinguishable from that of bilirubin. The reaction is further promoted by removing azobilirubin (reaction product) from the reaction system to shift the equilibrium of the system. For this reason, azobilirubin (reaction product between bilirubin and a diazonium compound) is sequentially transferred from the reagent layer to the registration layer so as to promote the reaction rate between the unreacted bilirubin and the diazonium compound. As a result, the desired color-changing reaction is remarkably accelerated, so that the period for analytical procedure is reduced and analytical accuracy is further enhanced.

The above-described registration layer can be made of a known hydrophilic colloid, but the registration layer is preferably a porous layer like the aforementioned reagent layer, if the reaction system involves a high molecular weight reaction product or a hydrophobic reaction product.

The porous registration layer is preferably made of a porous matrix composed of a binder and a particulate solid. As the binder, a known hydrophilic polymer can be employed. However, if the reaction product is positively or negatively charged, an electrically charged polymer having electric charge opposite to that of the reaction product is preferably employed as the binder of the registration layer, because the transfer of the above-described reaction product from the reagent layer to the registration layer can be accelerated by using such polymer. As the particulate solid employable for the formation of the above-described porous registration layer, a low light-reflective, particulate solid as mentioned hereinbefore such as microcrystalline cellulose, particulate diatomaceous earth, particulate silica gel, zeolite fine powder, particulate cellulose fibrous material or polymer beads is preferably employed.

In the multilayer analytical element of the present invention, a variety of constitutes employed for the conventional multilayer analytical elements can be employed. For instance, the multilayer analytical element of the invention may comprise an additional layer such as a high-reflecting layer, a light-blocking layer and an adhesive layer, singly or in combination.

The present invention will be further illustrated by the following non-limiting examples and comparison examples.

Example 1
(A) Preparation of multilayer analytical element for albumin analysis
A multilayer analytical element for analysis of albumin comprising a laminated structure of a support, a porous reagent layer for albumin detection, a light-blocking layer and a porous spreading layer was produced using the following materials and coating solutions.

1. Support
Transparent polyethylene terephthalate film (thickness: 180 μm) provided with a gelatin subbing layer

2. Porous reagent layer for albumin detection

| Composition of coating solution for porous reagent layer | |
|---|---|
| Bromocresol green | 1.0 g |
| Latex solution of divinylbenzene-styrene-N-methyl-N-vinylbenzylpiperidinium chloride copolymer (5:45:50), (resin content: 10% by weight) | 20 g |
| Microcrystalline cellulose (mean particle size: approx. 6 µm) | 10 g |
| Surface active agent (nonylphenoxypolyglycerol) | 0.5 g |
| Citric acid | 5.0 g |
| Water | 200 g |

A coating solution containing the dye, binder (copolymer) and microcrystalline cellulose in the weight ratio of 1:2 as described above was prepared. The coating solution was applied onto the gelatin subbing layer of the support, and then dried to form a porous reagent layer for albumin detection (thickness: 10 µm).

3. Light-blocking layer.

| Composition of coating solution for light-blocking layer | |
|---|---|
| Fine powder of titanium dioxide | 10 g |
| Microcrystalline cellulose (mean particle size: approx. 6 µm) | 20 g |
| Aqueous methyl vinyl ether-maleic acid copolymer solution [1% MEK (methyl ethyl ketone) solution, inherent viscosity $[\eta]$ at 25°C 1.0—1.4], (resin content: 5% by weight) | 40 g |
| Water | 60 g |

A coating solution containing the above-described materials was prepared. The coating solution was then applied onto the porous reagent layer and then dried to form a light-blocking layer (thickness: 8 µm).

4. Porous spreading layer
A cellulose acetate membrane filter (mean pore size: 3.0 µm, thickness: 180 µm) impregnated with an aqueous solution containing 1.0 weight % of polyacrylamide and 0.05 weight % of nonylphenoxypolyglycerol was fixed onto the light-blocking layer under pressure, and then dried to form a porous spreading layer.

(B) Analysis of human albumin
In physiological salt solutions were individually dissolved human albumin in the amounts of 3% by weight, 4% by weight and 5% by weight to prepare liquid samples of human albumin. When 10 µl of the liquid samples was spotted onto the spreading layer of multilayer analytical element prepared as above, formation of blue color proportional to the concentration of albumin contained in each liquid sample was observed from the support side. The reflection density of color formation was measured by applying a light having a wavelength of 600 nm after six min. from the application of liquid sample. The results are set forth in Table 1.

Example 2
(A) Preparation of multilayer analytical element for albumin analysis
The procedure of Example 1 for the preparation of a porous spreading layer was repeated except that a cotton broadcloth was employed instead of a membrane filter to prepare a multilayer analytical element for analysis of albumin.

(B) Analysis of human albumin
A liquid sample of human albumin was prepared in the same manner as described in Example 1. When 10 µl. of the liquid sample was spotted onto the spreading layer of the multilayer analytical element prepared as above, formation of blue color proportional to the concentration of albumin contained in each liquid sample was observed from the support side. The reflection density of color formation was measured in the same manner as described in Example 1. The results are set forth in Table 1.

Example 3
(A) Preparation of multilayer analytical element for albumin analysis
The procedure of Example 1 was repeated except that the porous spreading layer of the element was further provided thereon with a mix-spinned broadcloth (cotton/polyethylene terephthalate=3/7; one surface of which was impregnated with a polyethyl acrylate latex solution having a resin content of 25% by weight) under pressure, to prepare a multilayer analytical element for analysis of albumin.

6

(B) Analysis of human albumin

A liquid sample of human albumin was prepared in the same manner as described in Example 1. When 10 µl of the liquid sample was spotted onto the broadcloth spreading layer of the element, formation of blue color proportional to the concentration of albumin contained in each liquid sample was observed from the support side. The reflection density of color formation was measured in the same manner as described in Example 1. The results are set forth in Table 1.

TABLE 1
Optical density by reflection photometry (wavelength: 600 nm)

| | Albumin concentration of liquid sample | | |
| --- | --- | --- | --- |
| | 3% by weight | 4% by weight | 5% by weight |
| Example 1 | 0.25 | 0.54 | 0.85 |
| Example 2 | 0.23 | 0.50 | 0.76 |
| Example 3 | 0.20 | 0.45 | 0.70 |

Example 4

With the multilayer analytical element prepared in Example 3, the analytical procedure was carried out using whole blood as the liquid sample in the same manner as in Example 3.

When whole blood was spotted onto the spreading layer of the multilayer analytical element, formation of blue color proportional to the concentration of albumin contained in blood plasma was observed from the support side. This indicates that red hemoglobin present in whole blood gives no substantial disturbance to the analysis of albumin when the above-described multilayer analytical element is employed.

Example 5

(A) Preparation of multilayer analytical element for albumin analysis

A multilayer analytical element for analysis of albumin comprising a laminated structure of a support an adhesive layer, a porous reagent layer for albumin detection, an adhesive layer and a porous spreading layer was produced using the following materials and coating solutions.

1. Support

Transparent polyethylene terephthalate film (thickness: 180 µm) provided with a gelatin subbing layer

2. Adhesive layer

| | |
| --- | --- |
| Deionized gelatin | 8 g. |
| Methyl vinyl ether-maleic acid copolymer (1% MEK solution, inherent viscosity [η] at 25°C=2.6—3.5) | 5 g. |
| Sulfosalicylic acid (amounts per 1 m² of support) | 0.1 g. |

3. Porous reagent layer for albumin detection

| (Composition of coating solution for porous reagent layer) | |
| --- | --- |
| Bromocresol green | 0.06 g. |
| Divinylbenzene-styrene-N-methyl-N-vinylbenzyl-piperidinium chloride copolymer (5:45:50) | 1.1 g. |
| Microcrystalline cellulose (mean particle size: approx. 6 µm) (amounts per 1 m² of support) | 30 g. |

4. Adhesive layer

| | |
| --- | --- |
| Polyacrylamide (mean polymerization degree: 18,000) (amounts per 1 m² of support) | 1 g. |

5. Porous spreading layer

Cellulose acetate membrane filter (mean pore size: 3.0 µm, thickness: 180 µm)

(B) Analysis of human albumin

In physiological salt solutions were individually dissolved human albumin in the amounts of 3% by weight, 4% by weight and 5% by weight to prepare liquid samples of human albumin. When 10 µl. of each liquid sample was spotted onto the spreading layer of the element (encased on a white plastic mount) prepared as above, blue color formation proportional to the concentration of albumin contained in each liquid sample was observed from the support side. The color formation was measured by reflection photometry by applying a light having a wavelength of 600 nm after 6 min. from the application of the liquid sample. The results are set forth in Table 2.

TABLE 2
Optical density by reflection photometry (wavelength: 600 nm)

| Albumin concentration of liquid sample (% by weight) | | | | |
|---|---|---|---|---|
| 0 | 1.6 | 2.8 | 5.5 | 8.3 |
| 0.407 | 0.522 | 0.623 | 0.844 | 0.969 |

Example 6
(A) Preparation of multilayer analytical element for bilirubin analysis

A multilayer analytical element for analysis of bilirubin comprising a laminated structure of a support, a registration layer for receiving reaction product, a porous reagent layer for bilirubin detection, and a porous spreading layer was produced using the following materials and coating solutions in the same manner as in Example 1.

1. Support

Transparent polyethylene terephthalate film (thickness: 180 μm) provided with a gelatin subbing layer

2. Registration layer

| Composition of coating solution for registration layer | |
|---|---|
| Latex solution of divinylbenzene-styrene-N-methyl-N-vinylbenzylpiperidinium chloride copolymer (5:45:50) (resin content: 10% by weight) | 100 g. |
| Microcrystalline cellulose (mean particle size: approx. 6 μm) | 50 g |
| Water | 100 g |

A coating solution containing a binder (copolymer) and microcrystalline cellulose in the weight ratio of 1:5 as described above was prepared. The coating solution was applied onto the gelatin subbing layer of the support, and then dried to form a porous registration layer (thickness: 15 μm).

3. Porous reagent layer for bilirubin detection

| Composition of coating solution for porous reagent layer | |
|---|---|
| p-Sulfobenzenediazonium 1,5-naphthalanedisulfonate | 20 g |
| Microcrystalline cellulose (mean particle size: approx. 6 μm) | 40 g |
| Methyl vinyl ether-maleic acid copolymer aqueous solution (1% MEK solution, inherent viscosity [η] at 25°C=1.0—1.4) (resin content: 5% by weight) | 40 g |
| Calcium acetate | 200 mg |
| Sodium dodecylsulfate | 10 mg |
| Water | 50 g |

A coating solution containing the diazonium compound, binder (copolymer) and microcrystalline cellulose in the weight ratio of 1:20 as described above was prepared. The coating solution was applied onto the registration layer, and then dried to form a porous reagent layer for bilirubin detection (thickness: 10 μm).

4. Porous spreading layer

A mix-spinned broadcloth (cotton/polyethylene terephthalate=3/7) subjected to glow discharge treatment according to a method described in Japanese Patent Provisional Publication No. 57(1982)—66359 was impregnated with a solution containing 2.0% by weight of polyacrylamide (mean polymerization degree: 18,000) and 0.1% by weight of nonylphenoxypolyglycerol. The so impregnated broadcloth was fixed onto the aforementioned porous reagent layer under pressure, and dried to form a porous spreading layer.

8

(B) Analysis of bilirubin

A physiological saline solution containing 6% of albumin was added to bilirubin control (produced by Dade Corp., bilirubin precipitation: 20 mg/dl) to prepare bilirubin standard liquids of 20, 10, 5 and 2 mg/dl. When 10 µl of the standard liquid was spotted onto the spreading layer prepared as above, color formation proportional to the concentration of bilirubin contained in each standard liquid was observed from the support side. The color formation was measured by reflection photometry by applying a light having a wavelength of 560 nm after 6 min from the application of the standard liquid. The results are set forth in Table 3.

TABLE 3
Optical density by reflection photometry (wavelength: 560 nm)

| Bilirubin concentration of standard liquid (mg/dl) | | | |
|---|---|---|---|
| 2 | 5 | 10 | 20 |
| 0.24 | 0.30 | 0.45 | 0.68 |

Example 7

(A) Preparation of multilayer analytical element for bilirubin analysis

A multilayer analytical element for analysis of bilirubin comprising a laminated structure of a support, a registration layer for receiving reaction product, a porous reagent layer for bilirubin detection, and a porous spreading layer was produced using the following materials and coating solutions in the same manner as in Example 6.

1. Support

Transparent polyethylene terephthalate film (thickness: 180 µm) provided with a gelatin subbing layer

2. Registration layer

Composition of coating solution for registration layer
| | |
|---|---|
| Divinylbenzene-styrene-N-methyl-N-vinylbenzyl-piperidinium chloride copolymer (5:45:50) | 4 g. |
| Deionized gelatin (amounts per 1 m² of support) | 5 g. |

3. Porous reagent layer for bilirubin detection

Composition of coating solution for porous reagent layer
| | |
|---|---|
| p-Sulfobenzenediazonium p-toluenesulfonate | 40 mg. |
| Microcrystalline cellulose (mean particle size: approx. 6 µm) | 15 g. |
| Methyl vinyl ether-maleic acid copolymer (1% MEK solution, inherent viscosity $[\eta]$ at 25°C=1.0—1.4) | 1 g. |
| 7-(2,3-Dihydroxypropyl)theophylline [479-18-5] | 10 g. |

4. Porous spreading layer

In the course of the formation of the above-described porous reagent layer for bilirubin detection, a cotton broadcloth was pressed onto the solution-coated layer immediately after the coating was complete to form a porous spreading layer.

(B) Analysis of bilirubin

A physiological saline solution containing 6% of albumin was added to a bilirubin control (VERSATOL PEDIATRIC: trade name, produced by Warner-Lambert Corp.) to prepare albumin standard liquids of 19.5, 10.4 and 1.5 mg./dl. When 10 µl. of the standard liquid was spotted onto the spreading layer as prepared above, color formation proportional to the concentration of bilirubin contained in each standard liquid was observed from the support side. The color formation was measured by reflection photometry by applying a light having a wavelength of 550 nm after 6 min. from the application of the standard liquid. The results are set forth in Table 4.

TABLE 4
Optical density by reflection photometry (wavelength: 550 nm)

| Bilirubin concentration of standard liquid (mg./dl.) | | | |
|---|---|---|---|
| 0 | 1.5 | 10.4 | 19.5 |
| 0.17 | 0.34 | 0.56 | 0.84 |

Example 8 and Comparison Example 1
(A) Preparation of multilayer analytical element for bilirubin analysis
    A multilayer analytical element for analysis of bilirubin comprising a laminated structure of a support, a porous reagent layer for bilirubin detection, and a porous spreading layer was produced using the following materials and coating solutions in the same manner as in Example 6.—Example 8

1. Support
    Transparent polyethylene terephthalate film (thickness: 180 µm) provided with a gelatin subbing layer

2. Porous reagent layer for bilirubin detection

| Composition of coating solution for porous reagent layer | |
|---|---|
| p-Sulfobenzenediazonium 1,5-naphthalanedisulfonate | 200 mg. |
| Microcrystalline cellulose (mean particle size: approx. 6 µm) | 2 g. |
| Methyl vinyl ether-maleic acid copolymer (1% MEK solution, inherent viscosity [η] at 25°C=1.0—1.4) | 200 mg. |

3. Porous spreading layer
    A cellulose acetate membrane filter (mean pore size: 3.0 µm, thickness: 180 µm) was wetted with steam. Thus wetted filter was fixed onto the above-described porous reagent layer for detecting bilirubin under pressure to form a porous spreading layer.

(A) Preparation of multilayer analytical element for bilirubin analysis
    The procedure of Example 8 for the preparation of a porous reagent layer for bilirubin detection was repeated except that no microcrystalline cellulose was added, to prepare a multilayer analytical element for analysis of bilirubin.—Comparison Example 1

(B) Analysis of bilirubin
    Dyphylline [479-18-5] was added to a bilirubin control (produced by Dade Corp., bilirubin precipitation: 20 mg./dl) to prepare a bilirubin standard liquid of 10 mg./dl. When 10 µl. of the liquid was spotted onto the spreading layers of multilayer analytical elements prepared in the above-described Example 8 and comparison Example 1, color formation was observed from the support side. The color formation was measured with a lapse of time after application of standard liquid through reflection photometry with a light having a wavelength of 550 nm. The results are illustrated in the form of Curve P (result of Example 8) and Curve Q (result of Comparison Example 1) in Fig. 1.
    As is apparent from the results, the multilayer analytical element having the porous reagent layer prepared in Example 8 can prominently and rapidly produce the aimed color formation as compared with the multilayer analytical element having a reagent layer of a conventionally hydrophilic colloid prepared in Comparison Example 1.

Example 9
(A) Preparation of multilayer analytical element for bilirubin analysis
    The procedure of Example 8 for the preparation of a porous reagent layer for bilirubin detection was repeated except that the microcrystalline cellulose was replaced with diatomaceous earth, to prepare a multilayer analytical element for bilirubin analysis.

(B) Analysis of bilirubin
    After spotting 10 µl. of the bilirubin standard liquid of 10 mg./dl. onto the above-described spreading layer of the multilayer analytical element, the color formation observed from the support side was measured with a lapse of time through reflection photometry by applying a light having a wavelength of 550 nm. in the same manner as described in Example 8. As a result, the same curve as curve (P) illustrated in Fig. 1 representing the result of Example 8 was obtained.

**Claims**

1. A multilayer analytical element for analysis of an analyte in a liquid sample comprising:
a porous spreading layer for spreading the liquid sample;
a porous reagent layer provided in fluid contact with said spreading layer which contains a reagent producing or changing color on reaction with the analyte; and
a liquid-impermeable, light-transmissive support supporting said reagent layer, characterized in that the porous reagent layer is a porous matrix comprising a binder containing a reagent produced or changing color on reaction with the analyte and a particulate solid.

2. The multilayer analytical element as claimed in claim 1, in which said particulate solid is selected from microcrystalline cellulose, diatomaceous earth, silica gel, particulate zeolite, particulate cellulose fibrous material and polymer beads.

3. A multilayer analytical element for analysis of analyte present in a liquid sample comprising:
a porous spreading layer for spreading the liquid sample;
a porous reagent layer provided in fluid contact with said spreading layer which contains a reagent producing or changing color on reaction with the analyte;
a registration layer provided in fluid contact with said reagent layer which receives a color reaction product or a color-changed reaction product produced by the reaction between the analyte and the reagent; and
a liquid-impermeable, light-transmissive support supporting said registration layer, characterized in that the porous reagent layer is a porous matrix comprising a binder containing a reagent producing or changing color on reaction with the analyte and a particulate solid.

4. The multilayer analytical element as claimed in claim 3, in which said registration layer is a porous matrix comprising a binder and a particulate solid.

5. The multilayer analytical element as claimed in any one of claims 3 and 4, in which said particulate solid is selected from microcrystalline cellulose, diatomaceous earth, silica gel, particulate zeolite, particulate cellulose fibrous material and polymer beads.

**Patentansprüche**

1. Vielschichtiges analytisches Element für die Analyse eines Analyten in einer flüssigen Probe aus:
einer porösen Ausbreitungsschicht für das Ausbreiten der flüssigen Probe;
einer porösen Reagensschicht, die in fluidem Kontakt mit der Ausbreitungsschicht vorhanden ist und welche ein Reagens enthält, das bei der Reaktion mit dem Analyten Farbe erzeugt oder seine Farbe ändert;
einem flüssigkeitsundurchlässigen lichtdurchlässigen Träger, der die Reagensschicht trägt, dadurch gekennzeichnet, daß die poröse Reagensschicht eine poröse Matrix ist, die ein Bindemittel, welches das Reagens, das bei der Reaktion mit dem Analyten seine Farbe ändert oder Farbe erzeugt, enthält, und einen teilchenförmigen Feststoff umfaßt.

2. Vielschichtiges analytisches Element nach Anspruch 1, dadurch gekennzeichnet, daß der teilchenförmige Feststoff ausgewählt wird unter mikrokristalliner Cellulose, Diatomenerde, Silicagel, teilchenförmigem Zeolith, teilchenförmigem Cellulose-Fasermaterial und Polymerperlen.

3. Vielschichtiges analytisches Element für die Analyse eines Analyten, der in einer flüssigen Probe vorhanden ist, welche
eine poröse Ausbreitungsschicht für das Ausbreiten der flüssigen Probe;
eine poröse Reagensschicht, die in fluidem Kontakt mit der Ausbreitungsschicht vorhanden ist und welche ein Reagens enthält, das bei der Reaktion mit dem Analyten Farbe erzeugt oder seine Farbe ändert;
eine Aufzeichnungsschicht, die in fluidem Kontakt mit der Reagensschicht vorhanden ist und welche das Farbreaktionsprodukt oder das Reaktionsprodukt, das seine Farbe geändert hat, das durch die Reaktion zwischen dem Analyten und dem Reagens gebildet wurde, aufnimmt; und
einen flüssigkeitsundurchlässigen lichtdurchlässigen Träger, der die Aufzeichnungsschicht trägt, umfaßt, dadurch gekennzeichnet, daß die poröse Reagensschicht eine poröse Matrix ist, die ein Bindemittel, welches bei der Reaktion mit dem Analyten Farbe erzeugt oder seine Farbe ändert, enthält, und einen teilchenförmigen Feststoff umfaßt.

4. Vielschichtiges analytisches Element nach Anspruch 3, dadurch gekennzeichnet, daß die Aufzeichnungsschicht eine poröse Matrix ist, die ein Bindemittel und einen teilchenförmigen Feststoff enthält.

5. Vielschichtiges analytisches Element nach einem der Ansprüche 3 und 4, dadurch gekennzeichnet, daß der teilchenförmige Feststoff ausgewählt wird unter mikrokristalliner Cellulose, Diatomenerde, Silicagel, teilchenförmigem Zeolith, teilchenförmigem Cellulose-Fasermaterial und Polymerperlen.

**Revendications**

1. Elément analytique à couches multiples pour l'analyse d'un composant dans un échantillon liquide comprenant:

— une couche de diffusion poreuse pour la diffusion de l'échantillon liquide,

— une couche de réactif poreuse—à condition qu'elle soit en contact par l'intermédiaire du liquide avec ladite couche de diffusion—qui contient un réactif produisant une réaction colorée ou changeant de coloration lorsqu'il réagit avec le composant à analyser, et

— un support imperméable aux liquides et transmettant la lumière portant ladite couche de réactif, caractérisé en ce que la couche de réactif poreuse est une matrice poreuse comprenant un liant contenant un réactif produisant une réaction colorée ou changeant de coloration lorsqu'il réagit avec le composant à analyser et un solide particulaire.

2. Elément analytique à couches multiples selon la revendication 1, caractérisé en ce que ledit solide particulaire est sélectionné parmi la cellulose microcristalline, la diatomite, le gel de silice, la zéolite particulaire, un matériel fibreux contenant de la cellulose particulaire et des chaînes de polymères.

3. Elément analytique à couches multiples pour l'analyse d'un composant présent dans un échantillon liquide comprenant:

— une couche de diffusion poreuse pour la diffusion de l'échantillon liquide,

— une couche de réactif poreuse-à condition qu'elle soit en contact par l'intermédiaire du liquide avec ladite couche de diffusion—qui contient un réactif produisant une réaction colorée ou changeant de coloration lorsqu'il réagit avec le composant à analyser,

— une couche d'enregistrement à condition qu'elle soit en contact par l'intermédiaire du liquide avec la couche de réactif—qui recueille le produit de la réaction colorée ou le produit du changement de coloration, issu de la réaction entre le composant à analyser et le réactif; et

— un support imperméable aux liquides et transmettant la lumière portant ladite couche d'enregistrement, caractérisé en ce que la couche de réactif poreuse est une matrice comprenant

. un liant contenant un réactif produisant une réaction colorée ou changeant de coloration lorsqu'il réagit avec le composant à analyser

. un solide particulaire.

4. Elément analytique à couches multiples selon la revendication 3, caractérisé en ce que ladite couche d'enregistrement est une matrice poreuse comprenant un liant et un solide particulaire.

5. Elément analytique à couches multiples selon l'une quelconque des revendications 3 et 4, caractérisé en ce que le solide particulaire est sélectionné parmi la cellulose microcristalline, la diatomite, le gel de silice, la zéolite particulaire, un matériel fibreux contenant de la cellulose particulaire et des chaînes de polymères.

# FIG.I